# EUROPEAN PATENT APPLICATION

(11) **EP 3 002 024 A1**
(43) Date of publication of application: **06.04.2016**
(21) Application number: 14811081.0
(22) Date of filing: 13.06.2014
(51) Int. Cl.: A61M 16/08

(54) **GAS TRANSMISSION TUBE AND MEDICAL EQUIPMENT HAVING SAME**

(30) Priority: 14.06.2013 CN 201310236798
(71) Applicant: Beijing Aeonmed Co., Ltd., Beijing 100070 (CN)
(72) Inventor: FU, Chao, Beijing 100070 (CN)
(74) Representative: Hoarton, Lloyd Douglas Charles
(86) International application number: PCT/CN2014/079857
(87) International publication number: WO 2014/198236

(57) **Abstract**

Disclosed are a gas transmission tube and medical equipment having the same. The gas transmission tube comprises a body (100), wherein a plurality of gas transmission passages (101) penetrating through the body (100) along the length direction of the body (100) are formed in the body (100), the plurality of gas transmission passages (101) form a plurality of gas transmission passage groups (110), the plurality of gas transmission passage groups (110) are arranged at intervals along the vertical direction, and the number of the gas transmission passages (101) included in one of the plurality of gas transmission passage groups (110) is not equal to the number of the gas transmission passages (101) included in the other one of the plurality of gas transmission passage groups (110).

## Description

The patent application claims priority of the Chinese Patent Application No. 201310236798.5 entitled Gas Transmission Tube and Medical Equipment Having Same, filed on June 14, 2013, by Beijing Aeonmed Co., Ltd., the disclosure of which is incorporated herein by reference in its entirety.

### Technical Field

The invention relates to the medical field, especially relates to a gas transmission tube and medical equipment having same.

### Background Art

The existing gas transmission tube has the defect of inconvenient installation.

### Summary of the Invention

The purpose of this invention is to at least solve one of the technical problems in the prior art. Therefore, one object of the invention is to provide a gas transmission tube.

Another object of the invention is to provide a medical equipment having the gas transmission tube.

In order to realize the above objects, according to an embodiment of a first aspect of the invention, there is provided a gas transmission tube. The gas transmission tube comprises a body having a plurality of gas transmission passages penetrating through the body along a length direction of the body are formed in the body, the plurality of gas transmission passages forming a plurality of gas transmission passage groups arranged at intervals along the vertical direction, and the quantity of the gas transmission passages contained in one of the plurality of gas transmission passage groups being unequal to the quantity of the gas transmission passages contained in the other one of the plurality of gas transmission passage groups.

For the gas transmission tube according to the embodiment of the invention, the plurality of gas transmission passages are integrated on the body, and the quantity of the gas transmission passages contained in one of the plurality of gas transmission passage groups is unequal to the quantity of the gas transmission passages contained in the other one of the plurality of gas transmission passage groups. In this way, the plurality of air outlet passages may be quickly communicated with the plurality of gas transmission passages; moreover, the gas transmission tube (the plurality of gas transmission passages) is connected to the plurality of air outlet passages and the plurality of air inlet passages in only one way. The gas transmission tube fails to be connected to the plurality of air outlet passages and the plurality of air inlet passages in case of spinning with a fixed angle to thereby ensure correctly installing the gas transmission tube. Therefore, the gas transmission tube according to the embodiment of the invention has the advantages of convenience in installation and high installation efficiency, as well as correct installation.

In addition, the gas transmission tube according to the embodiment of the invention also has the additional technical features as follows:
According to one embodiment of the invention, the quantities of the gas transmission passages contained in any two of gas transmission passage groups are unequal.

According to one embodiment of the invention, each gas transmission passage group comprises a plurality of gas transmission passages being spaced along the horizontal direction in each gas transmission passage group. Therefore, the structure of the gas transmission tube is more reasonable.

According to one embodiment of the invention, there are two gas transmission passage groups, thereof one comprises two gas transmission passages and the other one comprises three gas transmission passages.

According to one embodiment of the invention, the width of the lower part of the body is bigger than the width of the upper part of the body in the horizontal direction. Therefore, the gas transmission tube can be further avoided from being mistakenly installed.

According to one embodiment of the invention, there is an arc shape forming at the corner of the cross section of the body. Therefore, a user is facilitated to hold the gas transmission tube to obtain a better hand feeling.

According to one embodiment of the invention, there is a circular arc shape forming at the corner of the cross section of the body. Therefore, a user is facilitated to hold the gas transmission tube to obtain a better hand feeling.

According to the embodiments of a second aspect of the invention, there is provided a medical equipment. The medical equipment comprises an air outlet portion having a plurality of air outlet passages, an air inlet portion having a plurality of air inlet passages and a gas transportation tube being the gas transportation tube according to the first aspect of the invention, thereof the first ends of the plurality of gas transportation passages being correspondingly connected to the plurality of air outlet passages respectively and the second ends of the plurality of gas transportation passages being correspondingly connected to the plurality of air inlet passages respectively.

Through setting the gas transmission tube according to the embodiment of the invention, the medical equipment according to the embodiment of the invention has the advantages of convenience in installation and high installation efficiency, as well as correct installation, etc.

According to one embodiment of the invention, the medical equipment is a ventilator or an anesthesia machine.

The invention will present the additional aspects and advantages in the following description, and the partial additional aspects and advantages could become obvious in the following description or via the practice of the invention.

### Brief Description of the Drawings

The above and/or additional aspects and advantages of the invention will become obvious and easy to understand from the description of embodiments with reference to the following drawings, wherein:
Fig. 1 is a schematic structural diagram of a gas transmission tube according to embodiments of the invention;
Fig. 2 is a schematic structural diagram of a medical equipment according to the embodiments of the invention; and
Fig. 3 is a schematic structural diagram of the medical equipment according to the embodiments of the invention.

### Detailed Description of the Preferred Embodiments

Hereinafter, embodiments of the invention are described in detail, examples of which are shown in the drawings, wherein the same or similar labels represent the same or similar component or the component of the same or similar function. The following embodiments described with reference to the drawings are exemplary, which are aimed at explaining the invention rather than restricting the invention,

In the description of the invention, the technical words are "center", "width", "thickness", "upper", "lower", "front", "back", "left", "right", "up-down", "horizontal", "top", "bottom", "inner", "outer" et al. these words represent orientation or position relationship shown based on the drawings in order to describe the invention and simplify the description and not to indicate or imply that the indicated device or component must have a specific orientation or constructed and operated in a specific orientation. Therefore, it is not to be understood as a restriction for the invention. Also the technical words "first", "second" are only be used for a descriptive purpose, and are not to be understood to indicate or imply the relative importance or imply the number of the indicated technical features. Therefore, features defined with "first", "second" may include one or more of the features explicitly or implicitly. In the description of the invention, "multiple" means two or more than two, unless specifically defined otherwise.

In the invention, the technical words "install", "be linked to", "connect to", "fix" et al. should be understood in generalization, unless specifically defined otherwise. For example, these technical words are described as fixed joint or removable connection or the integration of the connection; or mechanical joint or electrical connection; or direct connection or indirect connection via the middle medium or internal connection between the two component. The skilled persons in the art can understand the specific meaning about these technical words in the invention according to the specific circumstance.

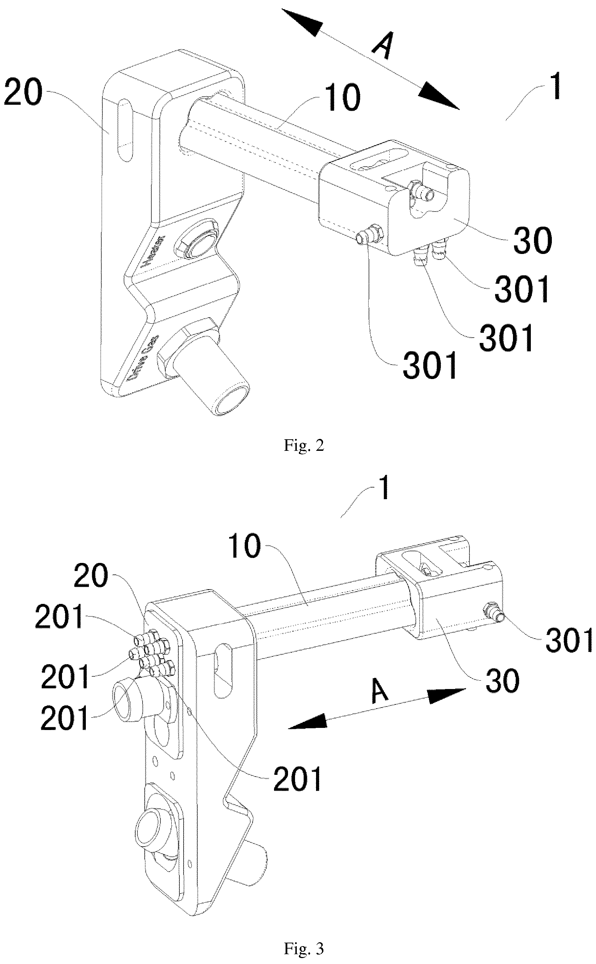

A medical equipment 1 according to the embodiments of the invention is described hereinafter with reference to Fig. 2 and Fig. 3.As shown in Fig. 2 and Fig. 3, the medical equipment 1 according to the embodiment of the invention includes a gas transmission tube 10, an air outlet portion 20 and an air inlet portion 30.

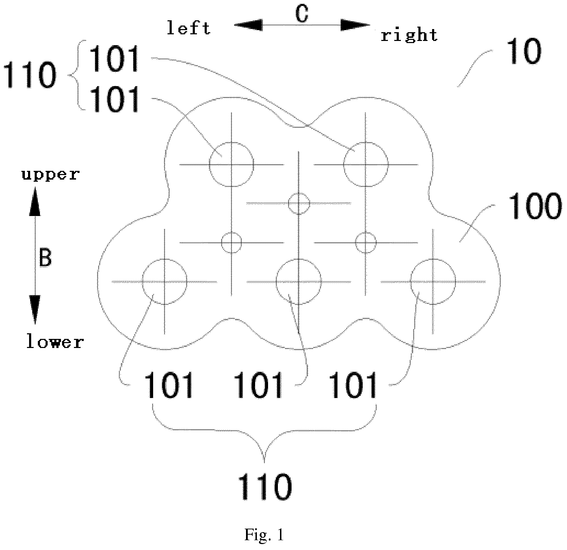

Firstly, the gas transmission tube 10 according to the embodiment of the invention is described with reference to Fig. 1 to Fig. 3. As shown in Fig. 1 to Fig. 3, the gas transmission tube 10 according to the embodiment of the invention includes a body 100, wherein a plurality of gas transmission passages 101 penetrating through the body along the length direction of the body 100 are formed in the body 100, the plurality of gas transmission passages 101 form a plurality of gas transmission passage groups 110, and the plurality of gas transmission passage groups 110 are spaced along the vertical direction. The quantity of the gas transmission passages 101 included in one of the plurality of gas transmission passage groups 110 is unequal to the quantity of the gas transmission passages 101 included in the other one of the plurality of gas transmission passage groups 110. The length direction of the body 100 is as shown by arrow A in Fig. 2 and Fig. 3 and the vertical direction thereof is as shown by arrow B in Fig. 1.

The air outlet portion 20 has internally a plurality of air outlet passages 201, and the air inlet portion 30 has internally a plurality of air inlet passages 301. The first ends of the plurality of gas transportation passages 101 are correspondingly connected to the plurality of air outlet passages 201 respectively, while the second ends of the plurality of gas transportation passages 101 are correspondingly connected to the plurality of air inlet passages 301 respectively. In other words, the quantity of the air outlet passages 201, the quantity of the air inlet passages 301 and the quantity of the gas transmission passages 101 may be equal; moreover, the first end of one gas transmission passage 101 is connected to one air outlet passage 201 and the second end of one gas transmission passage 101 is connected to one air inlet passage 301.

For the gas transmission tube 10 according to the embodiment of the invention, the plurality of gas transmission passages 101 are integrated on the body 100, and the quantity of the gas transmission passages 101 included in one of the plurality of gas transmission passage groups 110 is unequal to the quantity of the gas transmission passages 101 included in the other one of the plurality of gas transmission passage groups 110. In this way, the plurality of air outlet passages 201 may be quickly communicated with the plurality of gas transmission passages 101; moreover, the gas transmission tube 10 (the plurality of gas transmission passages 100) is connected with the plurality of air outlet passages 201 and the plurality of air inlet passages 301 in only one way. The gas transmission tube 10 fails to be connected with the plurality of air outlet passages 201 and the plurality of air inlet passages 301 in case of spinning with a fixed angle, thereby avoiding mistakenly installing the gas transmission tube 10.Therefore, the gas transmission tube 10 according to the embodiment of the invention has the advantages of convenience in installation and high installation efficiency, and avoid mistaken installation.

By setting the gas transmission tube 10 according to the embodiment of the invention, the medical equipment 1 according to the embodiment of the invention has the advantages of convenience in installation and high installation efficiency, as well as correct installation.

To be specific, the medical equipment 1 may be a ventilator or an anesthesia machine.

As shown in Fig. 1, in some embodiments of the invention, the quantities of the gas transmission passages 101 included in any two of gas transmission passage groups 110 are unequal. In other words, the quantity of the gas transmission passages 101 included in each gas transmission passage group 110 may be unequal to the quantity of the gas transmission passages 101 included in any other gas transmission passage group 110.

In one embodiment of the invention, as shown in Fig. 1, each gas transmission passage group 110 may include a plurality of gas transmission passages 101, and the plurality of gas transmission passages 101 of each gas transmission passage group 110 are spaced along the horizontal direction. Therefore, the structure of the gas transmission tube 10 is more reasonable. The horizontal direction is as shown by arrow C in Fig. 1.

As shown in Fig. 1, to be specific, two gas transmission passage groups 110 may be provided, one may include two gas transmission passages 101, and the other one may include three gas transmission passages 101.

Advantageously, as shown in Fig. 1, the width of the lower part of the body 100 may be bigger than the width of the upper part of the body 100 along the horizontal direction. Therefore, the gas transmission tube can be further avoided from being mistakenly installed.

As shown in Fig. 1, in one specific example of the invention, the corner of the cross section of the body 100 may be in an arc shape. Therefore, a user is facilitated to hold the gas transmission tube to obtain a better hand feeling.

To be specific, the corner of the cross section of the body 100 may be in a circular arc shape. Therefore, a user is facilitated to hold the gas transmission tube to obtain a better hand feeling.

In the description of the specification, references to the technical words "an embodiment", "some embodiments", "an example", "a specific example" or "some examples" et al. mean that specific features, structures, materials described in combination with the embodiment or example are embodied in at least one embodiment or example of the invention. In the specification, the schematic expressions of these technical words do not necessarily refer to the same embodiment or example. Furthermore, the described specific features, structures, materials or characteristics may be combined within any of one or more embodiments or examples in a suitable manner.

Although embodiments of the invention have been illustrated and described in the above, it is to be understood that the above mentioned embodiments are exemplary rather than the limitation of the invention. Those skilled in the art may make alterations, substitution and modifications to the above mentioned embodiments within the scope of the invention and without departing from the principle and gist of the invention.

## Claims

1. A gas transmission tube, wherein comprising a body, having a plurality of gas transmission passages penetrating through the body along a length direction of the body are formed in the body, the plurality of gas transmission passages forming a plurality of gas transmission passage groups arranged at intervals along the vertical direction, and the quantity of the gas transmission passages contained in one of the plurality of gas transmission passage groups being unequal to the quantity of the gas transmission passages contained in the other one of the plurality of gas transmission passage groups.

2. The gas transmission tube according to claim 1, wherein the quantities of the gas transmission passages contained in any two gas transmission passage groups are unequal.

3. The gas transmission tube according to claim 1, wherein each gas transmission passage group comprises a plurality of gas transmission passages being spaced along the horizontal direction in each gas transmission passage group.

4. The gas transmission tube according to claim 3, wherein thereof one comprises two gas transmission passages and the other one comprises three gas transmission passages.

5. The gas transmission tube according to claim 1, wherein the width of the lower part of the body is bigger than the width of the upper part of the body in the horizontal direction.

6. The gas transmission tube according to claim 1, wherein there is an arc shape forming at the corner of the cross section of the body.

7. The gas transmission tube according to claim 6, wherein the corner of the cross section of the body is in a circular arc shape. There is a circular arc shape forming at the corner of the cross section of the body.

8. A medical equipment, comprising wherein:
an air outlet portion having a plurality of air outlet passages,
an air inlet portion having a plurality of air inlet passages,
and a gas transportation tube being the gas transportation tube according to the first aspect of the invention, thereof the first ends of the plurality of gas transportation passages being correspondingly connected to the plurality of air outlet passages respectively and the second ends of the plurality of gas transportation passages being correspondingly connected to the plurality of air inlet passages respectively.

9. The medical equipment according to claim 8, wherein the medical equipment is a ventilator or an anesthesia machine.
